# EUROPEAN PATENT SPECIFICATION

(11) **EP 4 003 288 B1**
(45) Date of publication and mention of the grant of the patent: **06.05.2026**
(21) Application number: 20760391.1
(22) Date of filing: 29.07.2020
(51) Int. Cl.: A61K 8/73, A61K 8/65, A61Q 19/08, A61K 9/00, A61K 9/08, A61P 17/00

(54) **COSMETIC COMPOSITION WITH COMBINED FILLER AND SKIN REGENERATIVE EFFECT**
KOSMETISCHE ZUSAMMENSETZUNG MIT KOMBINATION AUS FÜLLSTOFF UND HAUTREGENERIERENDER WIRKUNG
COMPOSITION COSMÉTIQUE À CHARGE COMBINÉE ET EFFET RÉGÉNÉRATEUR DE LA PEAU

(30) Priority: 29.07.2019 WO PCT/EP2019/070361
(43) Date of publication of application: 01.06.2022
(73) Proprietor: Giuliani S.p.A., 20129 Milano (IT)
(72) Inventor: GIULIANI, Giammaria, 6926 Montagnola (CH); RINALDI, Fabio, 20129 Milano (IT); SPARAVIGNA, Adele, 20159 Milano (IT); MARZANI, Barbara, 27020 Carbonara Al Ticino (IT)
(74) Representative: Modiano, Micaela Nadia
(86) International application number: PCT/EP2020/071374
(87) International publication number: WO 2021/018950

(56) References cited:
- WO-A1-2013/036568
- WO-A1-2015/107237
- WO-A2-2009/024350
- WO-A2-2018/144093
- CN-A- 108 261 341

## Description

### FIELD OF THE INVENTION

The present invention relates to a dermo-cosmetic composition with combined filling and regenerative effect on skin.

The present invention origins in the cosmetic and aesthetic fields.

In particular, the present invention relates to a dermo-cosmetic composition with filler and regenerative effect on the skin, comprising the combination of a two-components dermal filler with amino acid components having biological activity on the skin.

### PRIOR ART

Human skin aging is a multifactorial, complex and chronic biological process affecting the main skin components.

Even if the skin aging mechanism is not yet fully know, recent studies have shown that the skin tends to lose the molecules responsible for binding and retaining water molecules while a reduction in the number and functionality of fibroblasts and collagen occurs.

The exposure of the skin to external factors such as solar radiation, heat or cold temperatures contributes to accelerate the aging of the skin.

Skin aging especially in the face of an individual manifests with a thinning of the epidermis and a loss of elasticity and with the appearance of wrinkles, furrows, atrophy, relaxation and loss of skin elasticity.

In the cosmetic field, preparations containing substances with moisturizing, emollient and regenerating action are commonly used to improve the skin appearance and remedy to the signs of skin aging.

However, the cosmetic effects resulting from the topical applications of these cosmetic substances is not considered fully satisfactory by the users.

For this reasons, certain techniques providing for the intradermal injection of dermo-cosmetic fillers have been developed in alternative to the topical application of cosmetic substances.

In aesthetic medicine, the dermal fillers are used to treat these signs of aging and to fill skin wrinkles or increase volumes.

Fillers are generally polymeric products injectable subcutaneously in the vicinity of wrinkles, skin furrows and in general in areas of the body where there is a sagging skin.

Once injected, they mainly carry out a mechanical action of filling the tissues, causing an action of stretching and relaxation of the skin which reduces the depth of the epidermal furrows.

The fillers available on the market can be divided into two main categories, those based on biodegradable substances and those non-biodegradable. Non-biodegradable fillers are called permanent fillers because they are not absorbable and are generally encapsulated in fibrous tissues and are not subject to hydrolysis or phagocytosis. While exerting a prolonged action, however, permanent fillers have a higher risk of developing allergic and rejection reactions by the human body.

Typical examples of permanent fillers include liquid silicones, silicone particles in suspension, particles of acrylic hydrogel crosslinked with hyaluronic acid, calcium hydroxyapatite, polymethylmethacrylate microspheres dispersed in collagen and formulations of polyacrylamide gel.

Biodegradable fillers are called temporary because their effect wears off over time and their application needs to be renewed to maintain unchanged the filling effect.

Examples of biodegradable fillers are disclosed in patent applications CN108261341 A, WO2009/024350 A2 or WO2013/036568 A1.

Biodegradable fillers are generally divided into two types, the intermediate duration ones and the long duration ones. The intermediate duration ones are absorbable after a few months of implantation and are made of materials subject to degradation by enzymatic hydrolysis, such as hyaluronic acid, and therefore they gradually lose the initial ability to fill and relax the tissues. Typically, these polymers are biodegradable materials present in nature, with high biocompatibility and affinity with water.

However, these polymeric matrices, such as hyaluronic acid, are rapidly hydrolysed once implanted in a tissue, and the results obtained, although appreciable from an aesthetic point of view, do not last long.

In order to remedy these problems, the biodegradable polymer matrix have been modified so as to obtain polymers in crosslinked form with longer duration of action.

At present, hyaluronic acids are available on the market which are treated with crosslinking agents so as to form covalent bonds which make the resulting polymer matrix less subject to enzymatic hydrolysis and enzymatic degradation, once injected into the tissues.

Because these crosslinked polymer matrices are less soluble and less prone to degradation, they behave as inert substances that play a mere tissue filling function, without making a contribution to improve the conditions of the tissues that have led to the formation or accelerated the formation of the wrinkle.

Some long duration of action fillers are also known which are made in synthetic polymer matrices. While having the advantage of being little biodegradable and thus having a long duration of action, these fillers are not free from drawbacks as they can cause allergic reactions or skin redness.

At present, there is still a demand for cosmetic products having an effective filling action combined with cosmetic properties.

One of the objects of the invention therefore is to provide a composition combining a filling effect with a biological effect on the skin.

A further object is to provide a dermocosmetic filler that, when injected in the skin, is almost free of side effects.

A further object of the invention is to provide an effective aesthetic or cosmetic method for the treatment of skin or photo-aging without using surgical treatments of the human body.

### SUMMARY OF THE INVENTION

The inventors, in accordance with an aspect of the invention, found that the combination of a dermal filler component with a selected peptide component having biological activity provides a combined filling and regenerative effect on the skin.

In one aspect, the invention provides a composition combining the filling properties typical of dermal fillers with the eutrophic and regenerative characteristics of peptides having biological and antiaging activity.

Typically, the composition of the invention contains a two-component filler that fill in or plump body areas that have lost volume and smoothness combined with a biologically active peptide component.

In accordance with a first aspect, the present invention provides a composition comprising a filler component in combination with a peptide having biological and antiaging activity on the skin, wherein the filler component comprises a not-crosslinked hyaluronic acid or a salt thereof and a recombinant type 1 collagen protein and wherein the peptide is a dipeptide selected from AlaPro, ProGly, GlyPro and mixtures thereof.

One of the two filler component is hyaluronic acid which is not crosslinked.

The other filler component is Recombinant type 1 Collagen protein.

The cosmetic anti-aging components includes peptides having biological activity whose use improves the appearance of skin.

The peptides having biological properties are dipeptides selected from AlaPro, ProGly, GlyPro and mixtures thereof. The composition of the invention may also contain additional amino acids or peptides.

Advantageously, the peptide having biological activity on the skin is a mixture of AlaPro, ProGly, GlyPro.

Typically, the peptide having biological activity on the skin stimulate the fibroblast activity combining the eutrophic and anti-aging action with the mechanical function of the filler component.

The composition of the invention finds application in the cosmetic and aesthetic field and in aesthetic medicine as well.

The composition of the invention is a cosmetic composition especially a dermal filler designed to treat signs of skin aging and/or photo-aging in a human beings.

The composition may be used with the following indication of use: plump up thinning lips, enhance or fill in shallow areas on the face, decrease or remove the shadow or wrinkle under the eyes caused by the lower eyelid, fill in or soften the look of recessed scars, fill in or soften static wrinkles, especially on the lower face treat Static wrinkles which usually are a result of a loss of collagen and elasticity in the skin.

Typically, the composition is a cosmetic composition suitable to treat or ameliorate the aspect of wrinkles especially around the mouth, thin lips, and cheeks.

The composition may also be used on forehead wrinkles, scars, and other areas that need extra volume for a smoother look.

The composition of the invention combines the mechanical function of a dermal filling with a cell regeneration activity and a delay in the skin aging process due to the sustained release of the peptides having biological activity to the tissues at the injection/implantation area.

Moreover, the combination of the fillers of the filling component increases the persistence time of the composition. For example, the composition exerts a combined filling and cosmetic effect lasting from 2 to 14 months, from 3 to 10 months, from 4 to 6 months.

Typically, the composition of the invention may be applied on the skin, preferably is an injectable dermal filler administered by intradermal or subcutaneous injection or implantation.

According to an aspect, the present invention provides a cosmetic method for treating skin aging and/or photo aging o comprising the injection of a cosmetically effective amount of a composition as defined herein.

According to certain embodiments, the cosmetic method of the invention stretches reduces, mitigates wrinkles, facial furrows, reshape, or fill the volumes of the skin.

In accordance with another aspect, the present invention provides a method for ameliorating an aesthetic aspect of the skin and/or fill the volume of skin by applying on skin or injecting in the subcutaneous layer of skin an aesthetically effective amount of a composition according to anyone of the embodiments disclosed herein.

The present invention is described in details herein below by making reference to the enclosed figures.

### BRIEF DESCRIPTION OF THE DRAWINGS

Some features and advantages of the present invention will become apparent from the accompanying drawings, in which:
Figs. 1A and 1B are photographic images made at times before (T0) and after 7 days (T7) from the skin treatment of a facial area of a female individual by intradermal injection of a composition having the formulation of Example 1;
Fig. 2 shows a bluish area in 3D image elaboration of the facial area shown in previous Figs. 1A, 1B showing a reduction of nasolabial folds and an increase in the volume on and nearby the areas of treatment.
Fig. 3 shows bar graphs illustrating the expression of the *AQUAPORIN 3* (*AQP3*) gene in PHENION full thickness models as determined by qRT-PCR according to Example 5;
Fig. 4 shows bar graphs illustrating the expression of the *COLLAGEN IV* (*COLIV*) gene in PHENION full thickness models as determined by qRT-PCR, as described in Example 5;
Fig. 5 shows bar graphs illustrating the expression of the *Cluster differentiation 44* (*CD44*) gene in PHENION full thickness models as determined by qRT-PCR, as described in Example 5.

### DETAILED DESCRIPTION OF THE INVENTION

The present invention originates from the finding that combining dermal filler components with a specific biologically active peptide component, a synergistic cosmetic structural effect is achieved on the skin affected by skin aging, photo aging.

In particular, the invention originates from the finding that the topical application or subcutaneous or intradermal injection of a combination composition combining a dermal component with mechanical and/or filler properties comprising a not-crosslinked hyaluronic acid or a salt thereof and a recombinant type 1 collagen protein with a biologically active peptide component selected from AlaPro, ProGly and GlyPro and mixture thereof, provides an effective and prolonged antiaging and filling action on the skin.

The prolongation of the antiaging action on the skin is due either to the extension of the filling effect and to the sustained release of the biologically active peptide component in the dermis where stimulates the production of collagen.

The application onto skin or the injection in the skin of the dermo-cosmetic composition increase the volume of the treated area determining the skin stretching and relaxation and a reduction of the depth of the epidermal corrugations.

Preferably the composition is a dermal filler.

The present invention provides either therapeutic or cosmetic uses of a composition as herein disclosed.

According to a first aspect thereof, the present invention therefore relates to a composition as defined in claim 1. Embodiments of the composition are defined in the appended dependent claims 2-7.

In another aspect, the use of a composition defined in claim 1 as dermal filler or to increase the volume of a soft tissue, especially skin, is disclosed herein.

Typically, for this use, the subcutaneous or intradermal injection of a cosmetically effective amount of the filler composition is provided.

A component of the composition is not-crosslinked hyaluronic acid (HA), a naturally occurring non-sulfate linear polysaccharide comprising repeating disaccharide units of d-glucuronic acid and *N*-acetyl-d-glucosamine linked by β-1-3 and β-1-4 glycosidic bonds. Hyaluronic acid is classified in three groups depending on its molecular weights: high molecular weight (HMWHA) is greater than 1 × 10⁶ Da, low molecular weight (LMWHA) is from 0.8 to 8 × 10⁵ Da, and oligo-HA is equal to or less than 6 × 10³ Da.

Hyaluronic acid is a primary component of the extracellular matrix of human connective tissues. It is also an important structural element in the skin.

The not-crosslinked hyaluronic acid of the composition may be in free form or as a salt in the form of a physiologically acceptable salt such as sodium or potassium hyaluronate.

A suitable not-crosslinked hyaluronic acid has a high molecular weight as defined above. For example, a suitable not-crosslinked hyaluronic acid may have a molecular weight greater than 1.000.000 Da, from 1.200.000, to 8.000.000 Da, from 2.000.000 to 4.000.000 Dalton.

According to certain embodiments the hyaluronic acid is not-crosslinked and has a molecular weight of 200 to 400kDa, for example 300kDa.

Typically, the molecular weight of not-crosslinked hyaluronic acid as described herein are weight average molecular weight MW, expressed in Dalton.

The not-crosslinked hyaluronic acid molecular weight may be determined using standard methods, for example as described by Ueno et al., 1988, Chem Pharm Bull. 36, 4971-4975; Wyatt 1993, Anal Chim Acta 272: 1-40; Watt Technologies 1999 "Light scattering University Dawn Course Manual and "Dawn Eos Manual" Wyatt Technology Corp. Santa Barbara CA (USA).

The not-crosslinked hyaluronic acid of the composition may be in the form of a gel which may be spread on the skin or administered by subcutaneous injection. According to some embodiments, the not-crosslinked hyaluronic acids of the invention have a crosslinking degree from 0.1 to 85%, from 1 to 50% 10 to 20% in relation to the molecular weight of the polymer.

A component of the composition is a recombinant type 1 collagen especially recombinant type I collagen α1 chain preferably produced by transgenic silkworms. Preferably, said transgenic silkworms possess an expression cassette in its genome and expresses a recombinant protein such as recombinant type 1 collagen in its middle silk gland. The expression cassette comprises a polynucleotide having the SEQ ID NO: 1 or a part thereof as described on pages 13 and 14 of EP 1 811 027 B1 which typically is linked with a structural gene for the recombinant protein.

Typically, the recombinant type 1 collagen used herein may be obtained by the method disclosed in the European Patent EP 1 811 027 B1 especially in par. [0031], [0032] or claims 16 and 17, or in its priority application JP 2004301834 filed on 15 October 2004, especially as disclosed under par. 0040.

For example, the human recombinant type 1 collagen is obtained by a process for producing a recombinant protein comprising extracting the recombinant protein from cocoons of a transgenic silkworm which possesses (in its genome) an expression cassette for expressing the recombinant protein in silkworm middle silk gland and secrete the recombinant protein in the sericin layer of silk filaments. A suitable polynucleotide comprises a polynucleotide corresponding to a promoter region of sericin 1 gene or sericin 2 gene which is functionally linked with a polynucleotide corresponding to baculovirus homologous regions especially having the nucleotide sequence of SEQ ID NO: 1 or a part thereof as reported either in EP 1 811 027 or JP 2004301834.

According to some embodiments, the composition of the invention contains recombinant type 1 collagen, especially human recombinant type 1 collagen, from 0,0001 to 5%, from 0,001 to 1,000% by weight

Typically, the composition of the invention is a composition in a form suitable for the application by subcutaneous or intradermal injection or by implantation in a body area, especially face, of a human being.

According to some embodiments, the composition of the invention contains from 0.01 to 80%, from 0.5 to 60%, from 1 to 50%, or from 10 to 40% by weight of not-crosslinked HA.

According to some embodiments, the composition of the invention contains from 0.001 to 20%, from 0.01 to 10%, from 0,5 to 4%, by weight of recombinant type 1 collagen.

A further component of the composition is a dipeptide selected from AlaPro, ProGly, GlyPro and especially mixtures thereof. The mixture of the three dipeptides is preferred to achieve a combined filler and regenerative effect on the skin.

The composition may also contain a biologically active amino acid performing a function in skin care and selected from Alanine, Valine, Glycine, Proline, OH-Proline, Aspartic acid, Lysine, Glutamic Acid, Glutamine, Asparagine, Leucine and mixtures thereof. Preferably, the amino acids Alanine, Valine, Glycine, Proline, OH-Proline, lysine are L- isomers.

Advantageously, the amino acids and peptides have a biological activity on the skin, especially on skin collagen metabolism and improve the collagen synthesis rate.

The amino acids of the composition maintain the skin's hydration in physiological conditions, strength the immune system thus providing an overall healthy appearance to the skin. They amino acid also protect skin from free-radical damage and activates the physiological trophism of skin thus reducing the signs of ageing.

The above biologically active amino acids and/or peptides activate the fibroblasts and are active on the intracellular components of the skin thus improving the aesthetic aspect of the skin. This cosmetic effect on the skin is complementary to the main filling effects exerted by the filling components of the composition.

In accordance with an embodiment the composition combines cosmetically effective amounts of
i) a filler component comprising or made of not-crosslinked hyaluronic acid and recombinant type 1 collagen with
ii) biologically active peptides AlaPro, ProGly, GlyPro.

The composition may contain a physiologically acceptable excipient or carrier. Typically, the physiologically acceptable carrier of the composition of the invention is an excipient, carrier or diluent suitable for intradermal or intracutaneous/intradermal administration or topical application. A suitable carrier may be water for the subcutaneous or intradermal.

Suitable carriers in liquid form include water, preferably ultrapure or for pharmaceutical use, isotonic saline solution (0.9%), aqueous solutions with phosphate buffer (PBS), Ringer's solutions, or aqueous solutions containing one or more of carbonate, citrate, acetate, glycine and mixtures thereof.

According to some embodiments, the composition of the invention contains one or more pH regulating agents and/or buffer systems of the conventional type in the techniques of preparation of injectable pharmaceutical solutions.

For example, phosphate or tartrate buffers may be used to adjust the pH to physiologically compatible values.

In certain embodiments the composition contains a NaOH solution to have a pH from 4.5 to 8, from 6 to 7.6.

Typically, the composition of the invention in injectable form is a sterile and pyrogen-free saline.

According to some embodiments, the composition of the invention is in the form of gel or hydrogel suitable for subcutaneous or intradermal injection.

In some embodiments, the gel or hydrogel particles have a particle size equal to or less than 700 µm. For example, from 50 to 700 µm, or from 100 to 400 µm, or from 200 to 300 µm, measured as the mass median diameter by laser diffraction technique or by microscopic analysis.

According to other embodiments, the monophasic gel is almost free from gel particles.

According to some embodiments, the composition of the invention is in the form of a water-based suspension.

According to some embodiments, the composition of the invention further comprises one or more physiologically acceptable excipients such as antioxidants, diluents, solvents, preservatives, bactericidal agents, stabilizers, emulsifiers, surfactants, buffers, humectants, dyes, or other excipients commonly used in cosmetic/pharmaceutical preparation techniques.

Suitable excipients are sodium chloride, sodium phosphate monobasic and or dibasic, cellulose and its derivatives such as carboxymethylcellulose. hydroxymethylcellulose, hydroxypropyl methylcellulose, methylcellulose, hydroxypropylcellulose, hydroxyethylcellulose, carboxyethylcellulose, ethylhydroxyethylcellulose, carboxymethylcellulose, cellulose acetate butyrate, cellulose acetate phthalate, and mixtures thereof.

The composition of the invention may further contain one or more antioxidants such sodium bisulfite, glutathione, coenzyme Q and mixtures thereof or vitamins such as vitamin B6, vitamin C.

In some embodiments, the composition of the invention comprises further cosmetically active substances which can be administered by injection.

In accordance with an aspect of the invention, a cosmetic or aesthetic treatment method is provided that comprises application of a cosmetically acceptable amount of a composition as defined herein.

The cosmetic or aesthetic treatment method of the invention may be applied in the treatment of any area of the body, such as the face, the lips, the area around the eyes, breasts, buttocks, and in particular to improve the aesthetic quality of a anatomical feature.

According to certain embodiments, the composition of the invention is a dermal filler or a combination composition suitable for use for aesthetic purposes, in particular to increase the volume of areas of the body, for example the lips, around the eyes, or in the treatment, prevention, distension of wrinkles, skin folds or corrugations, particularly those located on the face.

In some embodiments, the cosmetic treatment method involves the intradermal or subcutaneous injection of a composition according to any one of the embodiments previously described for filling wrinkles, facial corrugations, skin depressions or scars.

In certain embodiments, the composition of the invention may contain a local anesthetic, such as lidocaine, tetracaine, mepivacaine, procaine, benzocaine, and mixtures thereof, analgesics such as non-steroidal anti-inflammatory drugs, antimicrobials such as antibiotics for local use and mixtures thereof.

In some embodiments, the active principles contained in the composition of the present invention can be combined or mixed as active principles in intimate admixture with a suitable carrier and/or excipient according to pharmaceutical techniques.

Advantageously, the composition of the invention is a combination composition. According to some aspects of the present invention, the dermal filler or the filler composition of the invention can be applied in the field of aesthetic medicine.

In some embodiments, the dermal filler or the composition of the invention are applied in reconstructive surgery, such as in maxillofacial or aesthetic surgery, for example in plastic surgery or conservative aesthetics, for example in blepharoplasty or rhinoplasty.

In accordance with other aspects of the present invention, the filler or the filler composition of the invention can be applied in cosmetic and/or aesthetic filed.

In accordance with an aspect of the invention, a method for the treatment of an area of the body of an individual for aesthetic purposes is provided comprising the application or administration to an individual of a filler or of a composition comprising a filler according to the invention.

### Definitions

In the present document, the term "carrier" refers to an excipient, carrier, diluent or adjuvant which may be present in the composition of the invention. Any carrier and/or excipient suitable for the desired form of preparation for administration is envisaged in the uses described herein.

The term "cosmetically acceptable" as used herein means that the composition or components thereof are suitable for cosmetic use and when applied, do not cause toxicity, allergic response, redness, incompatibility, instability and similar undesired reactions.

The term "physiologically acceptable" means that the substance, when applied or administered to the human body, does not cause toxicity, allergic response, redness, incompatibility, instability and similar undesired reactions.

In general, the term "crosslinked" refers to intermolecular bonds that bind molecules or individual polymer chains such as HA or monomeric moieties thereof in a more stable structure.

Within the scope of invention, the term "monophasic gel" means a network of crosslinked viscoelastic gel comprising less than 20%, less than 10%, less than 5% by weight of gel particles.

The term biocompatible means a product or products or dermal filler that are physiologically acceptable and that when injected do not cause significant adverse reactions or anyway if they occur, they are of local type and are among the reactions and discomfort commonly encountered upon injection or implantation of products of known use.

The following examples are provided to illustrate the present invention.

### EXAMPLE 1

Cosmetic composition having the following formulation

| INGREDIENT | AMOUNT % W/W |
|---|---|
| WATER FOR INJECTIONS | q.b |
| Sodium Hyaluronate LW P-FIL | 0,01-2,000 |
| Sodium Carboxymethylcellulose MW | 0,01-4,000 |
| Human Recombinant Type 1 Collagen | 0,001-1,000 |
| ALA-PRO | 0,001-4,00 |
| PRO-GLY | 0,001-4,00 |
| GLY-PRO | 0,001-4,00 |
| L-ALANINE | 0,01-4,00 |
| L-VALINE | 0,01-4,00 |
| L-GLYCINE | 0,01-4,00 |
| L-PROLINE | 0,01-4,00 |
| L-OH-PROLINE | 0,01-4,00 |
| ASPARTIC ACID | 0,01-4,00 |
| LySINE HCL | 0,01-4,00 |
| GLUTAMIC ACID | 0,01-4,00 |
| GLUTAMINE | 0,01-4,00 |
| ASPARAGINE | 0,01-4,00 |
| LEUCINE | 0,01-4,00 |
| VITAMIN B6 | 0,01-3,500 |
| Soda Solution 30% | q.b to pH |
| SODIUM CHLORIDE | 0,01-1,00 |
| BIPHASIC SODIUM PHOSPHATE | 0,01-1,00 |
| MONOPHASIC SODIUM PHOSPHATE | 0,01-1,00 |
| | 100,000 |

### EXAMPLE 2

Composition having the following formulation

| **COMPOSITION** | **%** |
|---|---|
| Water ppi | 91,825 |
| Ala-pro | 0,001 |
| Pro-gly | 0,001 |
| Gly-pro | 0,001 |
| Alanine | 0,130 |
| Valine | 0,170 |
| Glycine | 0,150 |
| Proline | 0,090 |
| OH-Proline | 0,090 |
| Aspartic Acid | 0,150 |
| Lysine HCl | 0,100 |
| Glutamic Acid | 0,080 |
| Glutamine | 0,080 |
| Asparagine | 0,080 |
| Leucine | 0,030 |
| Vitamin B6 | 2,000 |
| Hyaluronic Acid p-fil Lw | 2,000 |
| Sodium Carboxymethylcellulose | 3,000 |
| Recombinant Type 1 Collagen | 0,022 |
| Biphasic Sodium Phoshate Dodecahydrate | 0,090 |
| Monophasic Sodium Phosphate Dihydrate | 0,017 |
| Sodium Chloride | 0,150 |
| Sodium Hydroxide Solution 30% | q.b pH 6,50-7,50 |

The above composition is obtained by a process including the following steps:
1) weigh the ppi water needed.
2) add under stirring, respectively: Sodium Chloride, Biphasic Sodium Phoshate Dodecahydrate, Monophasic Sodium Phosphate Dihydrate. Let dissolve.
3) once dissolved, add all the amino acids in the same order of the formula.
4) dissolve the amino acids and bring the pH in a range from 6,5 to 7,5. Bring the pH around 7,10
5) add the Vitamin B and let dissolve
6) add powdered Sodium Hyaluronate LW, as defined above, till complete dispersion
7) add recombinant Collagen till dispersion
8) add sodium Carboxymethylcellulose till dispersion and swelling overnight.

### EXAMPLE 3

Composition with the following formulation

| INGREDIENT | AMOUNT % W/W |
|---|---|
| WATER FOR INJECTIONS | q.b |
| Sodium Hyaluronate LW P-FIL | 0,01-2,000 |
| Sodium Carboxymethylcellulose MW | 0,01-4,000 |
| Recombinant Type 1 Collagen | 0,001-1,000 |
| ALA-PRO | 0,001-4,00 |
| PRO-GLY | 0,001-4,00 |
| GLY-PRO | 0,001-4,00 |
| Soda Solution 30% | q.b to pH |
| SODIUM CHLORIDE | 0,01-1,00 |
| BIPHASIC SODIUM PHOSPHATE | 0,01-1,00 |
| MONOPHASIC SODIUM PHOSPHATE | 0,01-1,00 |

### EXAMPLE 4

Experimental evidence of the cosmetic effect of the combined composition.

An open, clinical trial was performed on an individual of female gender of 60 years. The individual was affected by wrinkles on the face.

Two visits were performed: basal and 1 week after the injection.

The individual was treated by intradermal (subcutaneous) injection of a composition having the formulation of Example 1.

The intradermal injection was performed at time T0 (baseline) during the basal visit, after evaluations planned by the study procedure, in the malar area (zygomatic protuberance), by bolus technique using a needle. In the alternative, a cannula for aesthetic uses may be used for injecting the composition.

The amount of tested product injected, was determined by the investigator for each subject in 3 ml. This dosage may be varied from 2 to 5 ml.

The efficacy of the cosmetic effect was evaluated in a visit made 7 days (T7) after the day of the treatment.

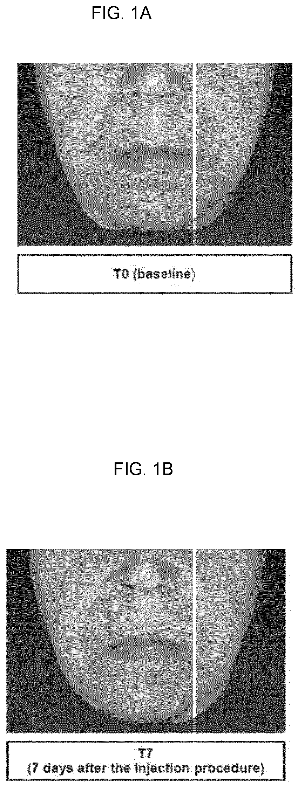

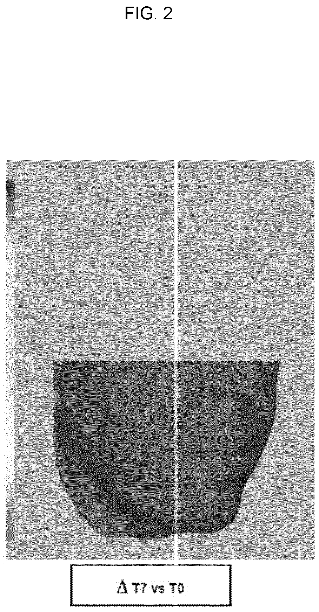

As can be seen in comparative Figures 1 and 2, after 7 days (T7) from the day T0 (baseline) of the cosmetic treatment, nasolabial folds were reduced and cheek volume increased (bluish area in 3D image elaboration). This outcome was confirmed by 3D image elaboration of the face of the individual made before and 7 days after the cosmetic treatment, as illustrated in Fig. 2.

### EXAMPLE 5

### 1. AIM

To check the in vitro effectiveness on skin restoration of different ingredients (Gly-Pro, Pro-Gly, Ala-Pro, recombinant type 1 collagen and not-crosslinked Hylauronic Acid) in single or in combination (combination composition).

Hereinbelow, the term "collagen" means recombinant type 1 collagen.

With the term "MIX AA" are meant the mixture of dipeptides AlaPro, ProGly and GlyPro.

### 2. MATERIALS

### 2.1 Samples

| **INNER NAME:** | **GLY-PRO** | **PRO-GLY** | **ALA-PRO** | **COLLAGEN Rec type 1** | **HYALURONIC ACID** |
|---|---|---|---|---|---|
| **LAB ID:** | MIX AA | MIX AA | MIX AA | C | HA |
| **LOT:** | BCBV7537 | BCBY9798 | BCBN4704V | MP061120CT | 18081311 |
| **DESCRIPTION:** | Sigma P0880-1g | Sigma: G3002-10G | Sigma A3263-1g | NeoSilk 54001 | PFIL LW |
| **STORAGE:** | RT. | RT | RT | RT | RT |
| **CONCENTRATION:** | 0.001% | 0.001% | 0.001% | 0.022% | 2% |

### 2.2 Reagents and instruments

| **REAGENTS** | **SUPPLIER** |
|---|---|
| Agarose (For routine use) | SIGMA, A9539-100G |
| PHENION FULL THIKNESS MODELS | PHENION |
| ALI MEDIUM | PHENION |
| Dulbecco'sPhosphateBuffered Saline | SIGMA, D8537 |
| Ethidium bromide solution (10 mg/mL, for molecular biology, aqueous solution) | SIGMA, E1510 |
| Gel Loading Buffer | SIGMA, G2526 |
| RNAse, none detected | |
| PRIME SCRIPT RT reagent kit (Perfect Real time) | Takara |
| PreMix Ex Taq | TAKARA, RR039A |
| TaqMan^{®} Gene Expression Assays for GAPDH Hs99999905_m1 | APPLIED BYOSISTEMS, 4331183 |
| TaqMan^{®} Gene Expression Assays for AQP3 HS00185020_M1 | APPLIED BYOSISTEMS, 4331183 |
| TaqMan^{®} Gene Expression Assays for CD44 HS01075864 | APPLIED BYOSISTEMS, 4331183 |
| TaqMan^{®} Gene Expression Assays for COL4A1 HS00266237 | APPLIED BYOSISTEMS, 4331183 |

| **INSTRUMENTS** | **SUPPLIER** |
|---|---|
| QiaExpert | Qiagen |
| 15 L digital water bath from +5°C to + 100°C (Mod: Swbd1, BS-SWB2D) | Stuart |
| Balance (Mod. XS204) | Mettler Toledo |
| Laminar flow cabinet (Mod: Gemini) + UV lamp with anti-reflex equipment | SterilManifacturingDivision |
| HeraCell CO₂ incubator (Mod:150 ADV) | ThermoScientific |
| 85°C horizontal freezer ULT130, 120 L (Mod: Labfrost, MME-TE21140) | Elcold |
| Bürker counting chamber w/clamps (DI-DA-443/3) | Carlo Erba |
| Microplateautoreader (EL 808) | Biotek |
| Vortex | Arhos160-PBI International |
| MX3000p RT instrument | Stratagene |

### 2.3 Experimental model

3D Phenion^{®} full-thickness skin models were purchased from Henkel (Phenion^{®} FT; Düsseldorf, Germany) and cultured in small Petri dishes (3.5 cm in diameter) filled with 4 mL pre-warmed air-liquid-interface (ALI) medium, provided by the manufacturer. The medium was refreshed one time after an initial overnight equilibration period. Tissues were subjected to experiments after the overnight equilibration at 37 °C and 5% CO2.

### 3. Methods

After overnight equilibration 25uL of each sample was incubated on Phenion fullthikness insert for 24h, 37°C 5%CO₂.
- **Mix AA:** 0.001% GLY-PRO + 0.001% PRO-GLY+ 0.001% ALA-PRO
- **C:** 0.022% Collagen
- **HA:** 2% Hyaluronic Acid
- **C+HA:** 0.022% Collagen + 2% Hyaluronic Acid
- **MIX AA+ C:** 0.001% GLY-PRO + 0.001% PRO-GLY+ 0.001% ALA-PRO+ 0.022% Collagen
- **MIX AA + HA:** 0.001% GLY-PRO + 0.001% PRO-GLY+ 0.001% ALA-PRO + 2% Hyaluronic Acid
- **MIX AA + HA + COLLAGEN:** 0.001% GLY-PRO + 0.001% PRO-GLY+ 0.001% ALA-PRO + 2% Hyaluronic Acid+ 0.022% Collagen
- **Negative control:** 25uL ALI medium

Twenty-four hours after treatment RNA for qRT-PCR analysis was extracted. Tri Reagent (Sigma Aldrich) methods as described by Chomczynski and Mackey was used and the cDNA was then synthesized from 2µg RNA template in a 20µl reaction volume, using the PrimeScript RT-PCR Kit (Takara, Japan). The cDNA was amplified and detected by the Stratagene Mx3000P Real-Time PCR System (Agilent Technologies Italia S.p.A., Milan, Italy). The amplification of cDNA from NCTC cells was conducted using the following Taqman gene expression assays: HS00185020_M1 (aquaporin 3, AQP3), HS01075864 (Cluster differentiation 44, CD44), HS00266237 (Collagen IV, COLIV) and Hs99999905_m1 (human glyceraldehyde-3-phosphate dehydrogenase, GAPDH). GAPDH was used as housekeeping gene. PCR amplifications were carried out in a 20µl of total volume. The mixture of reaction contained 10µl of 2X Premix Ex Taq (Takara, Japan), 1µl of 20× TaqMan gene expression assay, 0.4 µl of RoX Reference Dye II (Takara, Japan), 4.6 µl of water and 4µl of DNA. PCR conditions were the following: 95°C for 30 sec followed by 40 cycles of 95°C for 5 sec, 60°C for 20 sec. PCR reactions were performed in duplicate using an MX3000p PCR machine (Stratagene, La Jolla, CA). Δ cycle threshold (26) was used for the calculation of the relative abundance in the expression of each gene.

### 4. RESULTS

| **Marker gene** | | **End point** |
|---|---|---|
| **Aquaporin 3** | AQP3 | Improved skin hydrobalance |
| **Collagen IV** | COLIVA1 | Efficacy collagen production |
| **Cluster differentiation 44** | CD44 | Efficacy HA homeostasis |

### AQP3, COLIV and CD44 were assayed as markers of skin restoration (La Gatta et al., 2016).

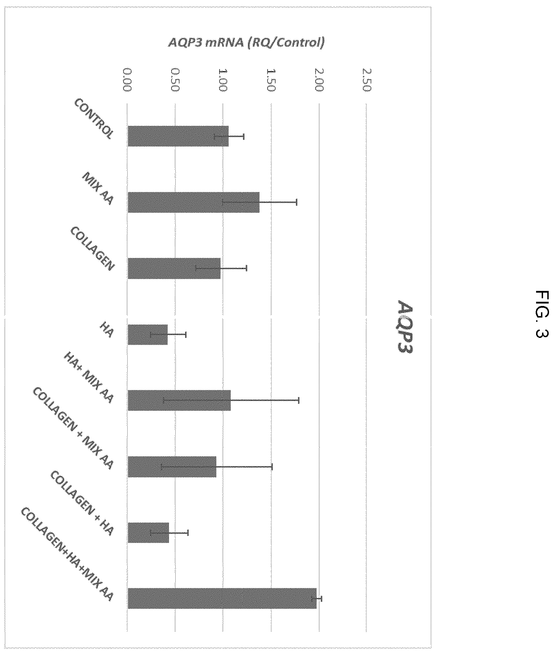

As evidenced in Figure 3, after 24h of incubation on Phenion full-thickness models HA, the mix of HA and COLLAGEN produced a significant (P<0.05) decrease of *AQP3* expression (Figure 3). On the contrary, MIX AA + HA + COLLAGEN produced a significant (p<0.01) effect on up-regulation of *AQP3.* No significant effect was reported for other tested compounds.

The data of Figure 3 evidence the expression of the AQUAPORIN 3 (AQP3) gene in PHENION full thickness models as determined by RT-PCR.

Inserts were treated with: medium alone (control); Mix AA (0.001% GLY-PRO + 0.001% PRO-GLY+ 0.001% ALA-PRO); COLLAGEN (0.022% Collagen); HA (2% Hyaluronic Acid); HA+ MIX AA (0.001% GLY-PRO + 0.001% PRO-GLY+ 0.001% ALA-PRO + 2% Hyaluronic Acid); COLLAGEN + MIX AA (0.001% GLY-PRO + 0.001% PRO-GLY+ 0.001% ALA-PRO+ 0.022% Collagen); COLLAGEN + HA (0.022% Collagen + 2% Hyaluronic Acid); COLLAGEN + HA+ MIX AA (0.001% GLY-PRO + 0.001% PRO-GLY+ 0.001% ALA-PRO + 2% Hyaluronic Acid+ 0.022% Collagen). Analyses were carried out after incubation at 37 ∘C for 24 h, under 5% CO2. Data are the means±SD of three separate experiments performed in triplicate. Statistical differences between mean values were determined with Tukey's HSD test. The asterisk indicates a significant difference (P < 0.05) with respect to the control.

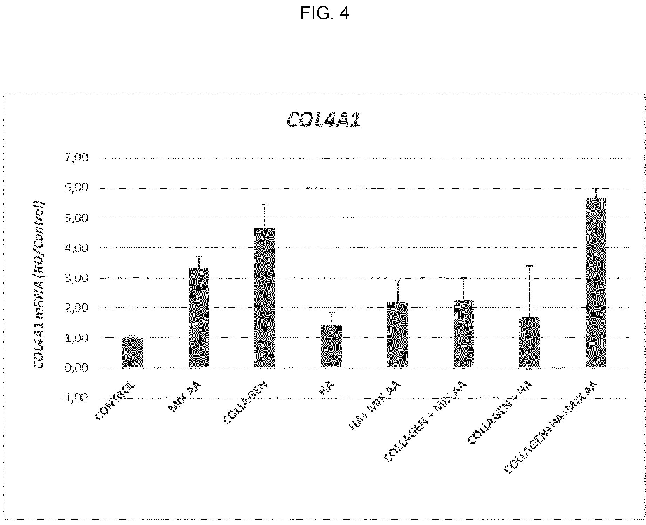

As shown in Figure 4, after 24h of incubation on Phenion fullthikness models HA, HA+ MIX AA and COLLAGEN + MIX AA produced a similar significant (p<0.05) increase of COL4A1 gene (Fig. 4).

The data of Figure 4 evidence that a synergistic effect was reported for the combination composition COLLAGEN + HA+ MIX AA which produced the highest increase (Fig. 4).

Inserts were treated with: medium alone (control); Mix AA (0.001% GLY-PRO + 0.001% PRO-GLY+ 0.001% ALA-PRO); COLLAGEN (0.022% Collagen); HA (2% Hyaluronic Acid); HA+ MIX AA (0.001% GLY-PRO + 0.001% PRO-GLY+ 0.001% ALA-PRO + 2% Hyaluronic Acid); COLLAGEN + MIX AA (0.001% GLY-PRO + 0.001% PRO-GLY+ 0.001% ALA-PRO+ 0.022% Collagen); COLLAGEN + HA (0.022% Collagen + 2% Hyaluronic Acid); COLLAGEN + HA+ MIX AA (0.001% GLY-PRO + 0.001% PRO-GLY+ 0.001% ALA-PRO + 2% Hyaluronic Acid+ 0.022% Collagen). Analyses were carried out after incubation at 37 ∘C for 24 h, under 5% CO2. Data are the means±SD of three separate experiments performed in triplicate. Statistical differences between mean values were determined with Tukey's HSD test. The asterisk indicates a significant difference (P < 0.05) with respect to the control.

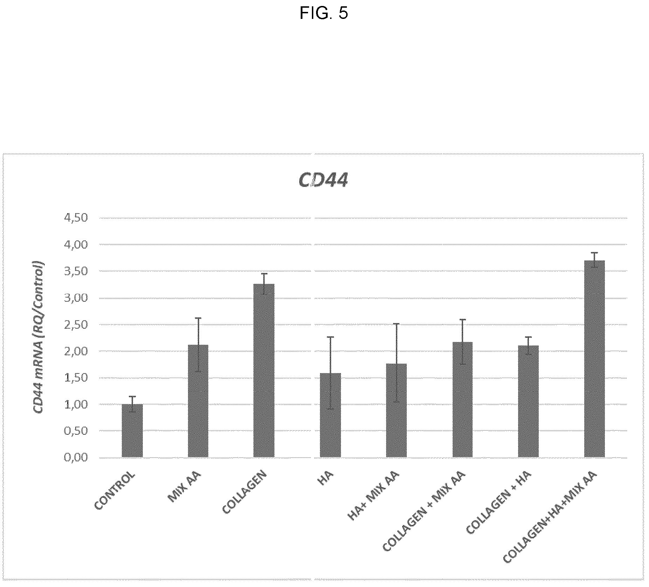

As shown in Figure 5, after 24h of incubation on Phenion fullthikness models a significant (p<0.01) and similar increase of CD44 gene was reported with samples MIX AA, COLLAGEN+MIX AA and COLLAGEN + HA (Fig. 5). The highest significant (p<0.005) increase was reported for the combination composition COLLAGEN + MIX AA + HA (Fig. 5).

Inserts were treated with: medium alone (control); Mix AA (0.001% GLY-PRO + 0.001% PRO-GLY+ 0.001% ALA-PRO); C (0.022% Collagen); HA (2% Hyaluronic Acid); HA+ MIX AA (0.001% GLY-PRO + 0.001% PRO-GLY+ 0.001% ALA-PRO + 2% Hyaluronic Acid); COLLAGEN + MIX AA (0.001% GLY-PRO + 0.001% PRO-GLY+ 0.001% ALA-PRO+ 0.022% Collagen); COLLAGEN + HA (0.022% Collagen + 2% Hyaluronic Acid); COLLAGEN + HA+ MIX AA (0.001% GLY-PRO + 0.001% PRO-GLY+ 0.001% ALA-PRO + 2% Hyaluronic Acid+ 0.022% Collagen). Analyses were carried out after incubation at 37 ∘C for 24 h, under 5% CO2. Data are the means±SD of three separate experiments performed in triplicate. Statistical differences between mean values were determined with Tukey's HSD test. The asterisk indicates a significant difference (P < 0.05) with respect to the control.

### 5. Conclusions

MIX AA + HA + COLLAGEN (combination composition with: 0.001% GLY-PRO + 0.001% PRO-GLY+ 0.001% ALA-PRO + 2% Hyaluronic Acid+ 0.022% Collagen) produced the highest significant effect on up-regulation of *AQP3, COLIV* and *CD44* genes.

### 6. REFERENCES

Chomczynski P, Mackey K. Modification of the TRI reagent procedure for isolation of RNA from polysaccharide- and proteoglycan-rich sources. Biotechniques 1995;19:942-5.

Vigetti D, Viola M, Karousou E, Rizzi M, Moretto P, Genasetti A, et al. Hyaluronan-CD44-ERK1/2 regulate human aortic smooth muscle cell motility during aging. J Biol Chem 2008;283:4448-58.

La Gatta A, De Rosa M, Frezza MA, Catalano C, Meloni M, Schiraldi C. Biophysical and biological characterization of a new line of hyaluronan-based dermal fillers: A scientific rationale to specific clinical indications. Mater Sci Eng C Mater Biol Appl. 2016;68:565-572. doi:10.1016/j.msec.2016.06.008.

### EXAMPLE 6

### A. STUDY OBJECTIVE

Primary end point of the study was to evaluate clinically and by non-invasive instrumental evaluations the safety and the aesthetic performance on the face of the dermal filler (combination composition of Example 5), containing : 0.001% GLY-PRO + 0.001% PRO-GLY+ 0.001% ALA-PRO + 2% Hyaluronic Acid + 0.022% type-1 recombinant collagen. Hyaluronic acid has a molecular weight of 300kDa.

One micro-injection session with the study product was performed by a specialized dermatologist, using bolus technique, on the face (zygomatic protuberance) of 20 female subjects with midface volume laxity due to aging mechanism, who met the inclusion and exclusion criteria required by the study procedure.

Secondary endpoints were aesthetic performance duration, Investigator's and subjects' satisfaction efficacy degree.

### B. SUMMARY OF THE STUDY METHODOLOGY

Four visits were performed during the trial: a basal visit, two intermediate visits (7 and 30 days after the injection procedure) and a final visit (90 days after the injection procedure). The assessment of the aesthetic result was established through the use of qualitative (clinical evaluations of cheek ptosis, nasolabial folds and marionette lines according to the Facial Volume Loss Scale photographic scale and Wrinkle Severity Rating Scale) and quantitative assessments (superficial and deep skin hydration, wrinkles profilometry, face volume image analysis), supported by photographic documentation.

All evaluations were carried out, at each study time, mono-laterally on the right or left face side according to a subjects' randomization list defined by the investigator before the subjects' inclusion.

### C. STUDY POPULATION

The study was conducted on 20 female volunteers, aged 40-66 years (mean = 52), with midface volume laxity, whose informed consent had been obtained. No male subject was included in the trial.

### D. RESULTS

No drop out or important event which may have interfered to the test results occurred during the study period, then the statistical analysis was performed on 20 included cases, who completed the trial as protocol directed.

The data processing took into consideration the comparison of each time versus basal conditions (T0) and was performed in accordance to our internal procedures (descriptive and inferential analysis) as follows:
- ***Clinical data:*** non-parametric test (Friedman test), followed in case of statistically significant result by Holm-Sidak Adjusted Wilcoxon signed rank test.
- ***Instrumental data:*** non-parametric test (Friedman test) when the normality hypothesis was rejected by the Shapiro-Wilk normality test (threshold at 5%) or parametric test (Anova test for repeated measures), when the normality hypothesis was confirmed, followed in case of statistically significant result by Holm-Sidak Adjusted tests (for details see "Statistical analysis summary tables").

Moreover the long lasting activity of the study treatment was expressed comparing the image analysis results on face volume obtained at T7 to the ones obtained at T30 and T90 using the statistical tests explained above for the instrumental data analysis.

### D.1. Efficacy evaluation

### D.1.1. Clinical assessment

The study treatment determined starting from T7 (see also 3D photographic documentation):
- a very significant improvement of ***cheek volume*** corresponding to a reduction of the FVLS mean value of 21.2% at T7 and 24.2% at T30 and T90 and a decrease of the clinical score of at least 1 grade (see FVLS photographic scale) respectively in 70%, 80% and 75% of included subjects;
- a statistically important reduction of ***wrinkles severity*** of 18.2% at T7 and 21.2% at T30 and T90, corresponding to a reduction of the clinical score of at least 1 grade respectively on 65%, 70% and 65% of volunteers.

### D.1.2. Instrumental evaluations

### D.1.2.1. Skin profilometry

Image analysis of the nasolabial folds and marionette lines confirmed the clinical assessment results; in fact, starting from T7, an important "anti-wrinkles" activity of the injective treatment (see also "PRIMOS photographic documentation") was showed.

More precisely for ***the nasolabial folds*** a clinically and statistically significant reduction of all profilometric parameters versus baseline was highlighted (see table below), index that the nasolabial folds was generally less visible and deep.

**(*) Holm-Sidak Adjusted Wilcoxon signed rank/t test p<0.05 vs T0**

| | ***VARIATION (%) versus T0*** | | |
|---|---|---|---|
| ***NASOLABIAL FOLDS*** | **T7** | **T30** | **T90** |
| **Ra** (average roughness of the analyzed profile) | -15.1% (*) | -27.5% (*) | -16.9% (*) |
| **Rt** (wrinkles total height) | -18.1% (*) | -25.5% (*) | -14.5% (*) |
| **Rv** (wrinkles maximum depth) | -21.3% (*) | -25.1% (*) | -14% (*) |

For the ***marionette lines*** the reduction percentage of Ra, Rt and Rv parameters were generally less marked, but still clinically/statistically significant vs T0 (see table below).

| | ***VARIATION (%) versus T0*** | | |
|---|---|---|---|
| ***MARIONETTE LINES*** | **T7** | **T30** | **T90** |
| **Ra** (average roughness of the analyzed profile) | -16.1% (*) | -25.2% (*) | -15.7% |
| **Rt** (wrinkles total height) | -14.9% | -21% (*) | -11.9% |
| **Rv** (wrinkles maximum depth) | -3.3% | -22.9% | -14.8% |

| | | | |
|---|---|---|---|
| **(*) Holm-Sidak Adjusted Wilcoxon signed rank test p<0.05 vs T0** | | | |

It is also important to note that, the anti-wrinkle effect on the nasolabial folds and marionette lines was more marked 30 days after the injection procedure; this result highlights an important activity of bio-stimulation mainly attributable to HA.

### D.1.2.2. Face volume image analysis

Face volume image analysis was carried on the 3D pictures taken at each study time by Vectra H1. In particular Vectra analysis module (VAM) merges and compares the images taken at two different times and automatically calculates the volume difference using a colour distance map. In order to calculate the volume variation induced by the study treatment, the volume measurement was performed for each subject mono-laterally on a selected area of the cheek and then analyzed by the software. When the merge was not applicable/evaluable, the data of the respective subjects were excluded from the evaluation.

Obtained results highlighted an average increase of volume versus T0 of 1.036 cc at T7, 0.964 cc at T30 and 0.917 cc at T90 (see graph and raw data table "3D face volume images analysis").

In particular at T7 75% of subjects (see table below) presented a very marked-marked improvement of face volume (35% of subjects with a cheek volume increase > 1 cc and 40% of subjects between 0.5 cc and 1 cc); although at T30 and T90 a trend and progressive reduction of cheek volume mean value was highlighted (-7% Δ_{T30-T0 vs} Δ_{T7-T0} and -11.5% Δ_{T90-T0 vs} Δ_{T7-T0}), the differences versus T7 were not statistically significant; moreover the high volumizing performance of the tested treatment was detectable in a more marked percentage of treated cases (90% at T30 and 85% at T90 - see table below). These results confirm the long lasting and persistent re-volumizing activity of the tested product.

| | **SUBJECTS %** | | |
|---|---|---|---|
| **VOLUME VARIATION** | **T7** | **T30** | **T90** |
| very marked improvement (Δ > 1 cc) | 35% | 40% | 40% |
| marked improvement (1 cc ≤ Δ > 0,5 cc) | 40% | 50% | 45% |
| light improvement (0,5 cc ≤ Δ > 0,2 cc) | 20% | 5% | 10% |
| unchanged ( Δ ≤ 0,2 cc) | 5% | 5% | 5% |

### D.1.2.3. Skin hydration

Measurements of skin hydration were performed:
- on skin surface by Corneometer CM825
- on deep skin layers at 0.5 mm and 1.5 mm of depth by MoistureMeterD.

Obtained results (see tables below) highlighted the important moisturizing efficacy of the tested treatment, started at T7 with the clinically/statistically significant increase of the superficial and deep skin layers hydration at 0.5 mm of depth.

| | ***VARIATION (%) versus T0*** | | |
|---|---|---|---|
| ***PARAMETER*** | **T7** | **T30** | **T90** |
| ***SUPERFICIAL SKIN HYDRATION*** | +14.8% | + 16.6% (*) | +17.8% (*) |
| ***DEEP SKIN HYDRATION at* 0.5 mm of depth** | +15.2% (*) | +15.6% (*) | +16.1% (*) |
| ***DEEP SKIN HYDRATION at* 1.5 mm of depth** | +3.2% | +7% | +4.3% |

| | | | |
|---|---|---|---|
| **(*) Holm-Sidak Adjusted Wilcoxon signed rank test p<0.05 vs T0** | | | |

Although no significant variation of deep hydration at 1.5 mm of depth was found at any study time, it is important to note at T30 a trend increase of the measured parameter.

### D.1.4. Efficacy evaluation by the volunteers

At the end of the trial (T90) each volunteer filled in a questionnaire regarding treatment efficacy and tolerance.

Considering the sum of medium, marked and very marked judgements, it's evident the subjects' positive judgement on the aesthetic performance of the study treatment (see table below).

| | **Sum of medium, marked and very marked judgements** |
|---|---|
| **Reduction of deep wrinkles** | 60% |
| **Reduction of superficial wrinkles** | 65% |
| **Lifting effect** | 60% |
| **Reshaping of face silhouette** | 65% |
| **Improvement of skin suppleness** | 70% |
| **Improvement of skin smoothness** | 65% |
| **Improvement of skin brightness** | 75% |
| **Improvement of skin hydration** | 80% |

### E. CONCLUSIONS

Obtained results confirm the anti-aging performance and the lasting effect of the tested dermal filler on midface defects due to aging mechanism. In fact the tested dermal filler thanks to a formulation based on recombinant collagen and low molecular weight hyaluronic acid (HA) determined starting from T7:
- a statistically significant improvement of FVLS score and face cheek volume ***(bio-revolumetric effect),***
- a statistically significant reduction of WSRS score ***(filling efficacy)**,*
- a clinically/statistically significant reduction of all profilometric parameters index of an ***anti-wrinkles efficacy*** (nasolabial folds and marionette lines appeared generally less visible),
- a clinically/statistically significant improvement of superficial and deep (at 0.5 mm of depth) skin hydration ***(moisturizing activity)**.*

As confirmed also by the profilometry and 3D-face volume image analysis results, the aesthetic performance of the tested dermal filler (combination composition) resulted generally more marked at T30, sign of a significant activity on dermal connective tissue and on epidermis able to provide new tonicity and consistency to the skin ***(bio-revitalization effect).***

The filler tolerance was judged good/excellent, in fact no unexpected adverse reaction related to the injection procedure occurred during the trial.

## Claims

1. A composition comprising a dermal filler component in combination with a peptide having biological and antiaging activity on the skin, wherein the filler component comprises a not-crosslinked hyaluronic acid or a salt thereof and a recombinant type 1 collagen protein wherein the peptide is a dipeptide selected from AlaPro, ProGly, GlyPro and mixtures thereof.

2. The composition according to claim 1 which is or is contained in a dermal filler.

3. The composition of claim 1 or 2 wherein the peptides are a mixture of AlaPro, ProGly and GlyPro.

4. The composition according to anyone of claims 1-3 further comprising an amino acid selected from Alanine, Valine, Glycine, Proline, OH-Proline, Lysine, Glutamic Acid, Glutamine, Asparagine, Leucine and mixtures thereof.

5. The composition according to anyone of claims 1-3 comprising the amino acid Alanine, Valine, Glycine, Aspartic acid, Proline, OH-Proline, Lysine, Glutamic Acid, Glutamine, Asparagine, Leucine.

6. The composition according to anyone of claims 1-5 wherein the recombinant type 1 collagen is the recombinant type I collagen α1 chain,
preferably produced by transgenic silkworms.

7. The composition according to anyone of claim 1-6 wherein the hyaluronic acid is from 200 to 400kDa.

8. Cosmetic use of a composition according to anyone of claims 1-7 for improving an aesthetic aspect of an individual.

9. Cosmetic use according to claim 8 for increasing the volume or reshaping a soft tissue, especially skin of the face or neck.

10. Cosmetic use according to claim 8 for reducing the depth or ameliorating the aesthetic aspect of wrinkles, folds, corrugations, furrows, folds, in particular on the face or neck.

11. Composition comprising a dermal filler component in combination with a peptide having biological and antiaging activity on the skin, wherein the filler component comprises a not-crosslinked hyaluronic acid or a salt thereof and a recombinant type 1 collagen protein and the peptide is selected from AlaPro, ProGly, GlyPro and mixtures thereof for use in the treatment of wrinkles, folds, corrugations, furrows, by a subcutaneous injection of a cosmetically active amount of said composition.

## Patentansprüche

1. Eine Zusammensetzung, die eine dermale Füllstoffkomponente in Kombination mit einem Peptid mit biologischer und Antiaging-Wirkung auf die Haut umfasst, wobei die Füllstoffkomponente eine nicht quervernetzte Hyaluronsäure oder ein Salz davon und ein rekombinantes Kollagenprotein vom Typ 1 umfasst, wobei das Peptid ein Dipeptid ist, gewählt aus AlaPro, ProGly, GlyPro und Mischungen davon.

2. Die Zusammensetzung gemäß Anspruch 1, die ein dermaler Füllstoff oder in einem dermalen Füllstoff enthalten ist.

3. Die Zusammensetzung gemäß Anspruch 1 oder 2, wobei die Peptide eine Mischung aus AlaPro, ProGly und GlyPro sind.

4. Die Zusammensetzung gemäß einem beliebigen der Ansprüche 1-3, die weiter eine Aminosäure umfasst, gewählt aus Alanin, Valin, Glycin, Prolin, OH-Prolin, Lysin, Glutaminsäure, Glutamin, Asparagin, Leucin und Mischungen davon.

5. Die Zusammensetzung gemäß einem beliebigen der Ansprüche 1-3, die die Aminosäure Alanin, Valin, Glycin, Asparaginsäure, Prolin, OH-Prolin, Lysin, Glutaminsäure, Glutamin, Asparagin, Leucin umfasst.

6. Die Zusammensetzung gemäß einem beliebigen der Ansprüche 1-5, wobei das rekombinante Kollagen vom Typ 1 die rekombinante Kollagen a1-Kette vom Typ 1 ist, vorzugsweise hergestellt von transgenen Seidenraupen.

7. Die Zusammensetzung gemäß einem beliebigen der Ansprüche 1-6, wobei die Hyaluronsäure von 200 bis 400kDa ist.

8. Kosmetische Verwendung einer Zusammensetzung gemäß einem beliebigen der Ansprüche 1-7 zur Verbesserung eines ästhetischen Aspekts eines Individuums.

9. Kosmetische Verwendung gemäß Anspruch 8 zur Erhöhung des Volumens oder Neuformung eines Weichgewebes, insbesondere Haut im Gesicht oder am Hals.

10. Kosmetische Verwendung gemäß Anspruch 8 zur Reduzierung der Tiefe oder Verbesserung des Aussehens von Hautfältchen, Falten, Runzelungen, Furchen, Falten, insbesondere im Gesicht oder am Hals.

11. Zusammensetzung, die eine dermale Füllstoffkomponente in Kombination mit einem Peptid mit biologischer und Antiaging-Wirkung auf die Haut umfasst, wobei die Füllstoffkomponente eine nicht quervernetzte Hyaluronsäure oder ein Salz davon und ein rekombinantes Kollagenprotein vom Typ 1 umfasst und das Peptid gewählt ist aus AlaPro, ProGly, GlyPro und Mischungen davon, zur Verwendung in der Behandlung von Hautfältchen, Falten, Runzelungen, Furchen, durch subkutane Injektion einer kosmetisch wirksamen Menge der Zusammensetzung.

## Revendications

1. Une composition comprenant un composant de charge dermique associée à un peptide présentant une activité biologique et anti-âge sur la peau, dans laquelle la composent de charge comprend de l'acide hyaluronique non réticulé ou un sel de celui-ci et une protéine de collagène de type 1 recombinante, le peptide étant un dipeptide choisi parmi AlaPro, ProGly, GlyPro et leurs mélanges.

2. La composition selon la revendication 1 qui est ou est contenue dans une charge dermique.

3. La composition selon la revendication 1 ou 2, dans laquelle les peptides sont un mélange d' AlaPro, ProGly et GlyPro.

4. La composition selon l'une quelconque des revendications 1 à 3, comprenant outre un acide aminé choisi parmi l'alanine, la valine, la glycine, la proline, l'OH-proline, la lysine, l'acide glutamique, la glutamine, l'asparagine, la leucine et leurs mélanges.

5. La composition selon l'une quelconque des revendications 1 à 3, comprenant les acides aminés alanine, valine, glycine, acide aspartique, proline, OH-proline, lysine, acide glutamique, glutamine, asparagine et leucine.

6. La composition selon l'une quelconque des revendications 1 à 5, dans laquelle le collagène de type I recombinant est la chaîne a1 du collagène de type I recombinant, de préférence produite par des vers à soie transgéniques.

7. La composition selon l'une quelconque des revendications 1 à 6, dans laquelle l'acide hyaluronique a un poids moléculaire compris entre 200 et 400 kDa.

8. Utilisation cosmétique d'une composition selon l'une quelconque des revendications 1 à 7 pour améliorer l'aspect esthétique d'un individu.

9. Utilisation cosmétique selon la revendication 8 pour augmenter le volume ou remodeler un tissu mou, en particulier la peau du visage ou du cou.

10. Utilisation cosmétique selon la revendication 8 pour réduire la profondeur ou améliorer l'aspect esthétique des rides, plis, ondulations, sillons, plis, en particulier sur le visage ou le cou.

11. Composition comprenant un composant de charge dermique en combinaison avec un peptide ayant une activité biologique et anti-âge sur la peau, dans laquelle le composant de charge comprend un acide hyaluronique non réticulé ou un sel de celui-ci et une protéine de collagène de type 1 recombinante, et le peptide est choisi parmi AlaPro, ProGly, GlyPro et de leurs mélanges, destinée à être utilisée dans le traitement des rides, des plis, des ondulations, des sillons, par injection sous-cutanée d'une quantité cosmétiquement active de ladite composition.
